## Europäisches Patentamt
## European Patent Office
## Office européen des brevets

(11) Veröffentlichungsnummer: **0 434 173 A2**

# EUROPÄISCHE PATENTANMELDUNG

(21) Anmeldenummer: 90250303.6

(22) Anmeldetag: 04.12.90

(51) Int. Cl.5: **A61K 31/66**, A61K 31/675, A61K 31/40, A61K 31/13, A61K 31/135, A61K 31/445, A61K 31/47

Die Bezeichnung der Erfindung wurde geändert (Richtlinien für die Prüfung im EPA, A-III, 7.3).

(30) Priorität: 04.12.89 DE 3940410

(43) Veröffentlichungstag der Anmeldung:
26.06.91 Patentblatt 91/26

(84) Benannte Vertragsstaaten:
AT BE CH DE DK ES FR GB GR IT LI LU NL SE

(71) Anmelder: SCHERING AKTIENGESELLSCHAFT
Berlin und Bergkamen
Müllerstrasse 170/178 Postfach 65 03 11
W-1000 Berlin 65(DE)

(72) Erfinder: Turski, Lechoslaw, Dr.
Spandauer Strasse 54
W-1000 Berlin 20(DE)
Erfinder: Bressler, Karin
Hampsteadstrasse 23A
W-1000 Berlin 37(DE)
Erfinder: Rettig, Klaus-Jürgen
Sangestrasse 2
W-1000 Berlin 26(DE)
Erfinder: Löschmann, Peter-Andreas
Württembergallee 8
W-1000 Berlin 19(DE)
Erfinder: Wachtel, Helmut, Dr.
Suarezstrasse 22
W-1000 Berlin 19(DE)

(54) **Verwendung von NMDA-Rezeptor-Antagonisten zur Behandlung chronischer neurodegenerativer Erkrankungen.**

(57) Die neue Verwendung von Antagonisten des N-Methyl-D-Aspartat (NMDA)-Rezeptorkomplexes oder deren physiologisch verträglichen Salze als Arzneimittel zur Prävention und Behandlung chronischer neurodegenerativer Erkrankungen, sowie pharmazeutische Mittel, die diese Verbindungen enthalten, werden beschrieben.

EP 0 434 173 A2

## NEUE VERWENDUNG VON NMDA-REZEPTOR-ANTAGONISTEN

Die Erfindung betrifft die neue Verwendung von Antagonisten des N-Methyl-D-Aspartat (NMDA)-Rezeptorkomplexes oder deren physiologisch vertraglichen Salze als Arzneimittel zur Prävention und Behandlung chronischer neurodegenerativer Erkrankungen, sowie pharmazeutische Mittel, die diese Verbindungen enthalten.

Im Zentralnervensystem von Säugern einschließlich der Menschen sind hohe Konzentrationen von excitatorischen Aminosäuren (EAS) wie Glutamat (GLU) und Aspartat (ASP) (Fonnum, F. , J. Neurochem. 42 : 1-11, 1984) vorhanden. Für die excitatorischen Aminosäuren existieren verschiedene Rezeptoren, die entsprechend ihrer spezifischen Agonisten als N-Methyl-D-Aspartat (NMDA)-, Kainat (KA)-und Quisqualat (QUIS)-Rezeptor bezeichnet werden.

Der NMDA-Rezeptor ist der am besten charakterisierte Rezeptor für EAS im Gehirn und stellt eine komplexe Einheit dar, deren normale Funktion abhängig ist von dem dynamischen Gleichgewicht zwischen verschiedenen potenzierenden und inhibitorischen Faktoren. Von seiner Funktion werden unterschiedliche neurologische Krankheitsbilder beeinflußt.

So sind von NMDA-Antagonisten neben antikonvulsiven, anxiolytischen und muskelrelaxierenden Effekten wie sie bei Epilepsie, Angstzuständen und Spastizität auftreten, auch protektive Effekte auf Nervenzellschädigungen infolge Ischämie/Hypoxie (Simon. R.D. et al. , Science 226 850-852, 1984), Hypoglykämie (Wieloch, T. , Science 230: 681-683, 1985) und Epilepsie (Chai, D.W. et al., Y. Neurosci 8: 185-196, 1988) beschrieben worden.

Obwohl es Hinweise darauf gibt, daß bei chronischen neurodegenerativen Erkrankungen wie Morbus Huntington und Morbus Alzheimer (Greenamyre, J.T. et al. , Prog. Neuropsychopharmacol. Biol. Psychiat. 12: 412-430, 1988), amyotropher Lateralsklerose und olivoponto-cerebellärer Degeneration (Plaitakis, A. et al. , Ann. Neuro. 22: 575-579, 1987; Science 216: 193-196, 1982) excitatorische Aminosäuren in der Pathophysiologie von Bedeutung sind, konnte bisher nicht gefunden werden, daß spezifische Antagonisten von EAS eine präventive Wirkung auf die Degeneration von Gehirnzellen ausüben.

Vor diesem Hintergrund ist der erfindungsgemäße Befund überraschend, daß NMDA-Antagonisten die Degeneration dopaminerger Neuronen verhindern und somit wirksame präventive Arzneimittel mit neuroprotektiver Wirkung darstellen.

Zu chronischen neurodegenerativen Erkrankungen, die durch fortschreitenden Untergang vornehmlich dopaminerger Neurone in der Substantia nigra des Gehirns ausgelöst werden, gehört auch der Morbus Parkinson. Die klinischen Symptome des Morbus Parkinson werden durch Neurotoxine wie 1-Methyl-4-phenyl-1,2,3,6-tetrahydropyridin (MPTP) bzw. dessen neurotoxischen Metaboliten 1-Methyl-4-phenylpyridiniumion (MPP$^+$) hervorgerufen (Langston, Y. W. et al. , Science 219: 979-980, 1983).

Zum Nachweis der protectiven Wirkung der erfindungsgemäßen Verbindungen wurde der toxische Metabolit MPP$^+$ lokal in die Substantia nigra von Ratten injiziert und somit eine rasch fortschreitende Degeneration der Dopaminneuronen bewirkt. Die gleichzeitige lokale Verabreichung des selektiven NMDA-Rezeptorantagonisten 2-Amino-7-phosphonoheptansäure (AP-7) hebt den neurodegenerativen Effekt von MPP$^+$ auf die Dopaminneuronen nahezu vollständig auf (Tabelle 1).

**Tabelle 1:**

Präventive Wirkung von AP-7 in verschiedenen Dosen und zu verschiedenen Zeitpunkten nach lokaler Koadministration mit $MPP^+$ (0.025 μmol pro Seite) in die Substantia nigra pars compacta (SNC) von Ratten. AP-7 wurde in verschiedenen Dosen zusammen mit $MPP^+$ jeweils in die rechte SNC injiziert. Jeweils die linke SNC wurde nur mit $MPP^+$ behandelt und diente als entsprechende Läsionskontrolle. Zu verschiedenen Zeitpunkten nach lokaler Injektion wurden die Tiere dekapitiert, die Gehirne nach in-situ-Fixation entnommen und anhand von mit Kresylviolett gefärbten Hirnschnitten einer definierten Schnittebene (Koronarschnitt unmittelbar rostral der Injektionsstelle) die Anzahl intakter, typischer Dopaminneurone der SNC unter einem Lichtmiskrop ausgezählt. [x:$p < 0.05$; xx:$p < 0.01$; xxx:$p < 0.001$; t-Test vs. entsprechende Kontrolle].

| SNC | Behandlung | Zeitpunkt | n | Anzahl intakter Neurone (Mittelwert ± S.E.M.) |
|---|---|---|---|---|
| links | Vehikel | 4 h | 8 | 158 ± 7 |
| rechts | Vehikel | 4 h | | 161 ± 6 |
| links | $MPP^+$ | 4 h | 3 | 18 ± 4 |
| rechts | $MPP^+$ + AP-7 0.025 μmol | 4 h | | 26 ± 2 |
| links | $MPP^+$ | 4 h | 4 | 27 ± 7 |
| rechts | $MPP^+$ + AP-7 0.1 μmol | 4 h | | 91 ± 24 x |
| links | $MPP^+$ | 4 h | 9 | 30 ± 4 |
| rechts | $MPP^+$ + AP-7 0.25 μmol | 4 h | | 128 ± 10 xxx |
| links | $MPP^+$ | 8 h | 4 | 25 ± 8 |
| rechts | $MPP^+$ + AP-7 0.25 μmol | 8 h | | 79 ± 9 xx |
| links | $MPP^+$ | 24 h | 4 | 14 ± 6 |
| rechts | $MPP^+$ + AP-7 0.25 μmol | 24 h | | 66 ± 16 x |

Aufgrund der genannten Untersuchungen eignen sich Antagonisten des NMDA-Rezeptor-komplexes zur Prävention und Behandlung von chronischen, durch umschriebenen und gut definierten Neuronenverlust charakterisierten, neurodegenerativen Erkrankungen, beispielsweise zur Prävention der Degeneration dopaminerger Neurone beim Morbus Parkinson, cholinerger Neurone beim Morbus Alzheimer oder corticaler und spinaler Motoneurone bei amyotropher Lateralsklerose und olivoponto-cerebellärer Degeneration. Die neuroprotective Wirkung ist spezifisch für Antagonisten des NMDA-Rezeptorkomplexes.

Für diese neue Verwendung kommen beispielsweise die folgenden Verbindungen oder deren physiologisch verträgliche Salze in Betracht:

1. ) Kompetitive NMDA-Antagonisten wie beispielsweise 2-Amino-7-phosphonoheptansäure (AP-7) und Analoga; 3-((±)2-Carboxy-piperazin-4-yl)-propyl-1-phosphonsäure (CPP) und Analoga, cis-4-Phosphonomethyl-2-piperidincarbonsäure (CGS 19755)

2.) Nicht-kompetitive NMDA-Antagonisten wie beispielsweise (±) 10,11-Dihydro-5-methyl-5H-dibenzo-[a,d]-cyclohepten-5,10-imin (MK-801), Memantine und andere Amantadin-Analoga, Ketamin und Analoga, Ifenprodil und Analoga.

3. ) Antagonisten der Glycin-Bindungsstelle wie beispielsweise Kynurensäure und Analoga, 1-Hydroxy-3-amino-pyrrolidin-2-on (HA-966) und Analoga.

4.) Polyamin-Antagonisten wie beispielsweise Spermin und Spermidin

5.) Anticholinergika mit NMDA-antagonistischer Wirkung wie beispielsweise Biperiden, Trihexiphenidyl.

6. ) Hemmer der excitatorischen Aminosäuren-Synthese.

Insbesondere eignen sich erfindungsgemäß solche NMDA-Rezeptor-Antagonisten, die spezifisch auf die durch das Neurotoxin $MPP^+$ ausgelöste Degeneration der Dopaminneuronen präventiv wirken.

Die Erfindung umfaßt auch pharmazeutische Mittel, die die genannten Verbindungen enthalten, deren Herstellung sowie die Verwendung der erfindungsgemäßen Verbindungen zur Herstellung von Arzneimitteln, die zur Behandlung und Prophylaxe der vorstehend genannten Krankheiten eingesetzt werden. Die Arzneimittel werden nach an sich bekannten Verfahren hergestellt, indem man den Wirkstoff mit geeigneten Träger-, Hilfs- und/oder Zusatzstoffen in die Form eines pharmazeutischen Präparates bringt, das insbesondere für die enterale oder parenterale Applikation geeignet ist. Als Hilfsstoffe für die gewünschte Arzneimittelformulierung sind die dem Fachmann bekannten inerten organischen und anorganischen Trägermaterialien geeignet wie zum Beispiel Wasser, Gelatine, Gummi arabicum, Milchzucker, Stärke, Magnesiumstearat, Talk, pflanzliche Ole, Polyalkylenglykole usw. Gegebenenfalls können darüber hinaus Konservierungs-, Stabilisierungs-, Netzmittel, Emulgatoren oder Salze zur Veränderung des osmotischen Druckes oder Puffer enthalten sein.

Die pharmazeutischen Präparate können in fester Form zum Beispiel als Tabletten, Dragees, Suppositorien, Kapseln oder in flüssiger Form, zum Beispiel als Lösungen, Suspensionen oder Emulsionen vorliegen.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,01 - 5000 mg, wobei die Dosis als einmal zu verabreichende Einzeldosis oder unterteilt in 2 oder mehrere Tagesdosen gegeben werden kann. Für die genannten Substanztypen sind die folgenden Einzeldosisbereiche als geeignet anzusehen:

1)

Für kompetitive NMDA-Antagonisten wie AP-7: 10 - 500 mg, vorzugsweise 150 mg, wie CPP und Analoga: 1 - 250 mg, vorzugsweise 25 mg, wie CGS 19755: 0,1 - 100 mg, vorzugsweise 10 mg.

2)

Für nicht-kompetitive NMDA-Antagonisten wie MK-801: 0,01 - 10 mg, vorzugsweise 1 mg, wie Memantin und Analoga: 10 - 1000 mg, vorzugweise 100 mg, wie Ketamin und Analoga: 10 - 750 mg, vorzugsweise 100 mg, wie Ifenprodil und Analoga: 0,1 - 100 mg, vorzugsweise 10 mg.

3)

Für Antagonisten der Glycinbindungsstelle wie Kynurensäure und Analoga: 100 - 5000 mg, vorzugsweise 500 mg, wie HA-966 0,01 - 100 mg, vorzugsweise 5 mg.

4)

Für Polyamin-Antagonisten wie Spermin und Spermidin: 100 - 5000 mg, vorzugsweise 500 mg.

5)

Für Anticholinergika mit NMDA-antagonistischer Wirkung wie Biperiden: 0,1 - 50 mg, vorzugsweise 2 mg, wie Trihexiphenidyl 0,1 - 50 mg, vorzugsweise 2 mg.

Die erfindungsgemäß verwendeten Stoffe, die insbesondere als Antiparkinson-Mittel eingesetzt werden, können gegebenenfalls zusammen mit anderen herkömmlich verwendeten Arzneistoffen mit Antiparkinsonwirkung wie L-Dopa verwendet werden. Durch Kombination der erfindungsgemäßen Arzneimittel mit herkömmlichen dopaminsteigernden Antiparkinsonmitteln wird die zu verabfolgende Dosis des herkömmlichen Arzneimittels verringert.

**Ansprüche**

1. Verwendung von Antagonisten des N-Methyl-D-Aspartat (NMDA)-Rezeptorkomplexes oder deren physiolosich verträglichen Salze zur Herstellung eines Arzneimittels zur Prävention und Behandlung chronischer neurodegenerativer Erkrankungen.

2. Verwendung nach Anspruch 1 dadurch gekennzeichnet, daß die Degeneration von Dopaminneuronen gehemmt wird.

3. Verwendung nach Anspruch 1 oder 2 dadurch gekennzeichnet, daß die durch das Neurotoxin 1-Methyl-4-phenyl-pyridiniumion (MPP$^+$) ausgelöste Degeneration gehemmt wird.

4. Verwendung nach Anspruch 1 zur Prävention und Behandlung des Morbus Parkinson.

5. Pharmazeutisches Mittel enthaltend eine wirksame Menge eines Antagonisten des NMDA-Rezeptorkomplexes oder dessen physiologisch verträglichen Salzes zur Anwendung nach Anspruch 1-4.

6. Pharmazeutisches Mittel nach Anspruch 5 in Kombination mit dopaminsteigernden Arzneimitteln.

7. Verfahren zur Herstellung eines pharmazeutischen Mittels nach Anspruch 5 oder 6, dadurch gekennzeichnet, daß man den Wirkstoff mit den geeigneten Träger-, Hilfs- und/oder Zusatzstoffen in eine geeignete Darreichungsform bringt.